# EUROPEAN PATENT SPECIFICATION

(11) **EP 3 592 233 B1**
(45) Date of publication and mention of the grant of the patent: **09.09.2020**
(21) Application number: 18707906.6
(22) Date of filing: 27.02.2018
(51) Int. Cl.: A61B 5/15, A61B 5/157, A61B 5/145, A61B 10/00, A61B 5/155

(54) **ENHANCED SAMPLING USING APPLIED ENERGY**
VERBESSERTE ENTNAHME MIT ANGEWANDTER ENERGIE
PRÉLÈVEMENT AMÉLIORÉ À L'AIDE DE L'ÉNERGIE APPLIQUÉE

(30) Priority: 07.03.2017 EP 17159502
(43) Date of publication of application: 15.01.2020
(73) Proprietor: Koninklijke Philips N.V., 5656 AG Eindhoven (NL)
(72) Inventor: GERHARDT, Lutz, Christian, 5656 AE Eindhoven (NL); JOHNSON, Mark, Thomas, 5656 AE Eindhoven (NL); UZUNBAJAKAVA, Natallia, Eduardauna, 5656 AE Eindhoven (NL); WORTELBOER, Pippinus, Maarten, Robertus, 5656 AE Eindhoven (NL); GROB, Timon, Rutger, 5656 AE Eindhoven (NL); STAPERT, Hendrik, Roelof, 5656 AE Eindhoven (NL)
(74) Representative: Philips Intellectual Property & Standards
(86) International application number: PCT/EP2018/054743
(87) International publication number: WO 2018/162277

(56) References cited:
- WO-A1-2005/074161
- WO-A1-2013/175196
- WO-A1-2016/019250
- WO-A2-2009/098487
- US-A1- 2004 176 706
- US-A1- 2011 087 306
- US-A1- 2013 079 605

## Description

### FIELD OF THE INVENTION

The present disclosure is directed generally to healthcare. More particularly, but not exclusively, various techniques disclosed herein relate to improved sampling of various bioanalytes from bodily fluids using insertable and/or wearable devices.

### BACKGROUND OF THE INVENTION

Biomarkers and/or analytes (often referred to herein generally as "bioanalytes") sampled from living tissue may provide various information about a molecular composition of blood, plasma, interstitial fluid, sweat, lymph and other body fluids, providing information essential for clinical diagnosis. Collecting bioanalytes from living tissue-including from fluids such as interstitial fluid, blood, and lymph fluid-is typically performed in clinical settings using benchtop microfluidic devices.

As research progresses on device miniaturization and reducing power consumption, and as manufacturing processes improve, wearable/insertable devices are becoming more accepted in clinical settings, and home monitoring is becoming an increasingly important feature of healthcare systems. Wearable and/or insertable devices have traditionally been used to measure vital signs such as heart rate, respiration rate, temperature, oxygen saturation, and so forth. However, it is only recently that they have been used to measure bioanalytes. Technical challenges associated with wearable/insertable biomarker monitoring devices include capturing bioanalytes from tissue and delivering them to an analysis unit quickly and efficiently, and in sufficiently high concentrations. It has been suggested that non-mechanical forces such as electrophoresis, electroosmosis, thermal gradients, capillary forces, and/or convection may be used, in combination with mechanical forces such as fluid pressure, to generally induce enhanced flow of biological fluids towards and through all microneedles of a wearable device simultaneously. But as wearable/insertable devices become more complex, with increased capabilities to perform *in vivo* analysis (*e.g.,* of bioanalyte presence and/or detection), there is a need for more individual control of fluid flow towards and through subsets or even individual microneedles.

### SUMMARY OF THE INVENTION

The present disclosure is directed to methods and apparatus for improved sampling of various bioanalytes from bodily fluids using wearable and/or insertable devices. In various embodiments, a wearable/insertable device with a plurality of microneedles may be operated to apply various types of externally input energy (*e.g.,* electricity, heat, light, ultrasound, etc.) at selected subsets of the plurality of microneedles, including at selected individual microneedles in some instances, to induce a local bioanalyte diffusion gradient and subsequent flow (*e.g.,* perfusion) of biological fluids and/or of individual bioanalytes (*e.g.,* while not inducing flow of the carrying biological fluid) in sampling regions of the tissue, and/or to induce local bioanalyte diffusion gradient and subsequent flow through microchannels of the microneedles.

This applied energy may induce an increased flow rate of bodily fluids (and hence, an enhanced flow of one or more bioanalytes), *e.g.,* due to blood microcirculation, skin perfusion, interstitial fluid flow, and/or tissue swelling. Consequently, an obtained sample may be representative of bioanalyte concentration in a substantial tissue volume. By inducing increased flow of bodily fluids, it is possible to accelerate bioanalyte intake and/or increase concentrations and/or volume of one or more bioanalytes in the sampling region, thereby enhancing bioanalyte capture, which in turn facilitates bioanalyte analysis.

Generally, in one aspect, an apparatus for sampling one or more bioanalytes in tissue may include: a base having one or more conduits adapted to receive fluid extracted from the tissue; a plurality of microneedles fluidly coupled with the one or more conduits and adapted to be pierced into the tissue such that a tip of each microneedle is positioned in a respective sampling region of the tissue; a plurality of individually-controllable energy emitters; and logic operably coupled with the plurality of individually-controllable energy-emitters. In various embodiments, the logic may be adapted to operate a subset of the plurality of individually-controllable energy emitters to apply energy at a first subset of the plurality of microneedles to induce bioanalyte flow through tissue towards the first subset or a second subset of the plurality of microneedles, or through the first subset or a second subset of the plurality of microneedles.

In various embodiments, the applied energy may include electricity. In various embodiments, the electricity may be direct current or alternating current. In various embodiments, the electricity may induce iontophoresis through the first or second subset of the plurality of microneedles. In various embodiments, the electricity may induce iontophoresis in respective sampling regions towards the tips of the first or second subset of the plurality of microneedles. In various embodiments, the electricity may induce electrophoresis or dielectrophoresis through the first or second subset of the plurality of microneedles. In various embodiments, the electricity may induce electrophoresis or dielectrophoresis in respective sampling regions towards the tips of the first or second subset of the plurality of microneedles.

In various embodiments, at least one of the energy emitters may be adjacent a proximal end of a microneedle of the plurality of microneedles. In various embodiments, at least one of the energy emitters may be coupled to the tip of a microneedle of the plurality of microneedles. In various embodiments, the applied energy may include heat that induces one or more heat gradients towards or through the first or second subset of the plurality of microneedles. In various embodiments, the applied energy may include ultrasound. In various embodiments, the ultrasound may induce one or more heat diffusion gradients towards or through the first or second subset of the plurality of microneedles. In various embodiments, the applied energy may include radio-frequency energy.

In another aspect, a method may include: removably affixing a wearable or insertable device that includes a substrate and a plurality of microneedles to tissue of a patient, wherein the applying includes inserting the plurality of microneedles into the tissue; operating a plurality of individually-controllable energy emitters of the wearable device to apply energy at a first subset of the plurality of microneedles to induce bioanalyte flow towards or through the first subset or a second subset of the plurality of microneedles; receiving one or more fluids from the tissue through microchannels of the plurality of microneedles; and analyzing the one or more fluids to detect one or more bioanalytes.

As used herein, the terms "biomarker" and "bioanalyte" may refer to any physiological substance and/or structure that can be objectively measured to, for instance, predict the incidence of outcome and/or disease. Biomarkers can be used for various purposes. In screening, biomarkers identify the risk of developing a disease, *e.g.,* by grouping individuals based on an estimated risk to develop a disease. In diagnostics, biomarkers identify a disease. Prognostic biomarkers predict disease progression. Pharmacodynamic biomarkers mark a particular pharmacological response. Some biomarkers may be analyzed to monitor disease activity and clinical response to an intervention. Some biomarkers that are sometimes referred to as "severity" biomarkers may be used as surrogate endpoints in clinical trials. Biomarkers may come in numerous forms and/or groups, including but not limited to cytokines and interleukins, electrolytes, ketones, triglycerides, insulin, glucose, cortisol, vitamins, anti-oxidants, reactive oxygen species, markers for cancer and anti-cancer therapy, markers of specific medications, micro-ribonucleic acid (miRNA), and so forth. Other biomarkers may be described through this specification.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the disclosure.
Figs. 1A and 1B illustrate an example apparatus for enhanced bioanalyte sampling, in accordance with various embodiments.
Figs. 2, 3, 4, and 5 depict examples of how different types of energy may be applied in different manners to enhance bioanalyte sampling/analysis, in accordance with various embodiments.
Fig. 6 depicts an example of an insertable device configured with selected aspects of the present disclosure, in accordance with various embodiments.
Fig. 7 depicts an example method for sampling bioanalytes in tissue that may be practiced, for instance, using the various apparatus described herein.

### DETAILED DESCRIPTION OF EMBODIMENTS

Various biomarkers/analytes (or generally "bioanalytes") may have relationships with certain diseases or conditions. Monitoring these biomarkers may provide valuable information regarding disease state (*e.g.* presence/absence) or disease severity and progression. Non-limiting examples of relations between different biomarkers/analytes and diseases or conditions are provided in Table 1, which includes a general function (*e.g.* skin barrier) and provides examples of analytes that may be tested and conditions or diseases for which they may be relevant. In addition to those listed in Table 1, other non-limiting conditions and associated analytes include: dehydration and electrolytes; obesity and ketones, triglycerides, and insulin; asthma and blood parameters; wound management and wound moisture, pH, and bacterial composition on skin or at wound site; diabetes and glucose, insulin, and ketones; stress and cortisol; malnutrition and vitamins and electrolytes; drug abuse and the drug(s) of abuse; drug compliance and specific sensors for medications; cancer/cancer treatment and markers for therapy and recurrence; and the like.

**Table 1**

| **Parameter** | **Examples of Analytes** | **Disease or Condition** |
|---|---|---|
| Skin Barrier | • Lipids and ceramides in the stratum corneum and upper epidermis | • Eczema |
| | | • Bacteria and other infections |
| | • Natural Moisturizing Factors (NMF) in upper epidermis | • Decubitus |
| | | • Psoriasis |
| | • Keratin-family proteins | |
| | • Anti-microbial proteins (AMP) | |
| | • pH | |
| | • Bacterial composition | |
| Antioxidant | • Carotenoids, beta carotene | • UV-induced skin ageing |
| | • Vitamin D | • Skin cancer |
| | • Vitamin C | • Multiple sclerosis |
| | | • Depression |
| Oxidative Stress | • Free radicals | • UV-induced skin ageing |
| | • Reactive oxygen species (ROS) | • Skin cancer |
| | | • Etc. |
| | • Products of oxidation (e.g. lipid peroxides, DNA and nucleotides damage) | |
| | • Pyrimidine dimers | |
| | • Ketones | |
| Inflammation | • Interleukins | • Metabolic syndrome |
| | • Growth factors | • Psoriasis |
| | • Leptin | • Psoriatic arthritis |
| | • Resistin | • Crohn disease |
| | • Infiltration of immune cells - T cells, neutrophilic granulocytes (NG), mast cells | • Cardiovascular disease |
| | | • Chronic, non-healing wounds |
| | • Spongiosis (excess of interstitial fluid) | |
| | • Vasodilatation | |
| | • Metaloproteinases (MMP) | |
| | • Loose walls of blood vessels | |
| Hormones | • Cortisol | • Chronic inflammation |
| | • Adrenaline | • Ovulation |
| | • Estrogens | • Women's health |
| | • Testosterones | • Men's health |
| | • Insulin | • Diabetes |
| Blood composition, capillaries or larger vessels | • LDL cholesterol | • Diabetes |
| | • Electrolytes | • Cardiovascular disease |
| | • Glycated Hemoglobin | • Metabolic syndrome |
| | • Glucose | • Response to therapy |
| | • Lactate | • Drug compliance |

As noted above, wearable/insertable devices are believed to be becoming more accepted in clinical settings, and home monitoring is becoming an increasingly important feature of healthcare systems. Wearable and/or insertable devices have traditionally been used to measure vital signs such as heart rate, respiration rate, temperature, oxygen saturation, and so forth. However, they have not traditionally been used to measure bioanalytes. A challenge with using wearable/insertable devices is capturing bioanalytes from tissue and delivering them to an analysis unit quickly and efficiently. In view of the foregoing, various embodiments and implementations of the present disclosure are directed to wearable and/or (subcutaneously) insertable apparatuses that are configured to enhance *in vivo* sampling of bioanalytes.

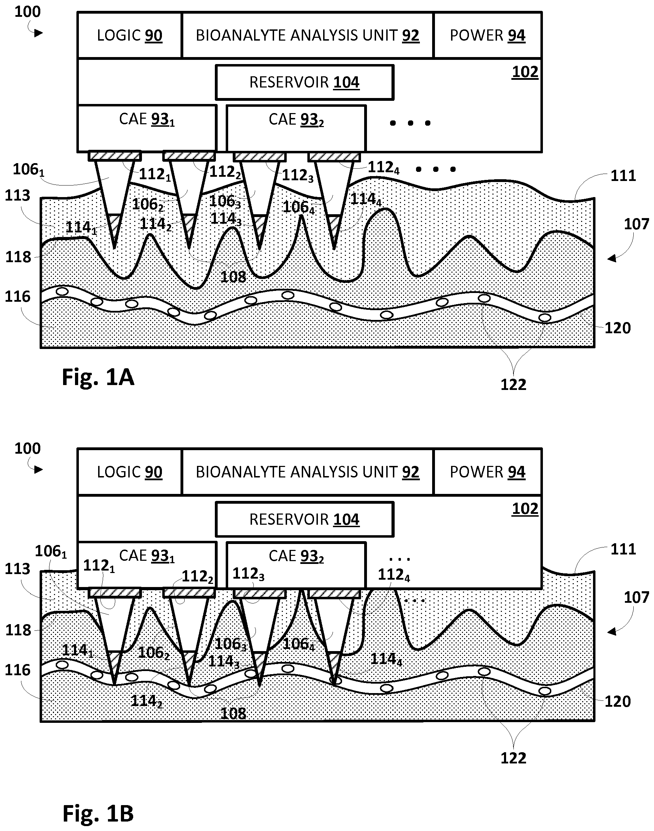

Referring to Figs. 1A-B, in one embodiment, an apparatus 100 for sampling one or more bioanalytes may include a device base 102 (or "substrate") having one or more reservoirs 104 to receive fluid extracted from living tissue 107, a plurality of microneedles 106 that are adapted to fluidly couple one or more reservoirs with tissue 107, and one or more core activation elements (CAE) 93 operable to cause one or more individually-controllable energy emitters 112, 114 to apply energy to subsets of the plurality of microneedles 106. In some embodiments, apparatus 100 may further include logic 90, a bioanalyte analysis unit 92, and a power unit 94. Logic 90 may take various forms, such one or more microprocessors that execute instructions stored in memory (not depicted), a field-programmable gate array (FPGA), an application-specific integrated circuit (ASIC), or other types of controllers and/or signal processors. In various embodiments, logic 90 may control various aspects of operation of apparatus 100 described herein, as will be described further below. In some embodiments, logic 90 may include one or more wired or wireless communication interfaces (not depicted) that may be used to exchange data (bioanalyte analysis results) with one or more remote computing devices using various technologies, such as Bluetooth, Wi-Fi, etc.

Bioanalyte analysis unit 92 may be operably coupled with logic 90 and may be configured to perform various types of analysis on fluids and/or bioanalytes contained in one or more reservoirs 104. Bioanalyte analysis unit 92 may provide various signals to logic 90 that pertain to various aspects of fluid and/or bioanalytes detected in reservoir 104, such as ratios of various bioanalytes, presence/amounts of various bioanalytes, and so forth. Power unit 94 may take various forms, such as one or more batteries, which may or may not be rechargeable, *e.g.,* using one or more integrated solar cells (not depicted), by periodically being connected to an external power source (*e.g.* via inductive coupling), or via other means (*e.g.,* harvesting various types of kinetic energy associated with movement of a wearer). Of course, in some embodiments, one or more of units 90, 92, and/or 94 may be omitted in favor of external computing resources, such as a computing device that may be operably coupled, for instance, with logic 90.

In various embodiments, the one or more reservoirs 104 may be fluidly coupled with one or more of the plurality of microneedles 106. Only four microneedles, 106₁₋₄, are depicted in Figs. 1A and 1B, but this is not meant to be limiting. As indicated by the ellipses to the right of microneedle 106₄, in various embodiments, any number of microneedles 106 may be included. In some embodiments, each microneedle 106 may include an inner lumen or microchannel (not depicted in Figs. 1A and 1B) that fluidly couples a tip 108 of the microneedle 106 (and hence, tissue 107 of the patient) with the one or more reservoirs 104. Various bodily fluids (*e.g.,* interstitial fluids, blood, sweat, etc.), which may contain a variety of selected bioanalytes, may pass through the microchannel from the tip 108 of the microneedle 106 to reservoir 104. Once in reservoir 104, the fluid sample(s) may be tested/assayed, *e.g.,* by bioanalyte analysis unit 92, for presence/quantity/ratios of various bioanalytes. Additionally or alternatively, in various embodiments, reservoir 104 may be transported, *e.g.,* as part of apparatus 100 or separated therefrom, to another location at which the fluid contained therein may be tested/assayed.

More generally, in various embodiments, one or more microneedles 106 may be fluidly coupled with one or more conduits or ports (not depicted). In embodiments with integral reservoir(s) 104, the conduit(s)/port(s) may take the form of a fluid coupling interface to the reservoir(s) 104. In embodiments without integrated reservoirs, microneedles 106 may be selectively fluidly coupled with fluid lines and/or removable reservoirs via the aforementioned conduits/ports.

In some embodiments, all or parts (*e.g.,* the tips 108) of microneedles (*e.g.,* 106) described herein may be constructed with biodegradable materials. For example, if tips (*e.g.,* 108) of the microneedles are biodegradable and sharp, they may be used to initially pierce the tissue, and then may dissolve so that they won't later break. Additionally, in some embodiments, the inner microchannel of a microneedle 106 may not necessarily extend all the way through the sharp biodegradable tip (which may instead be solid). Accordingly, the sharp tip may be used initially to pierce the tissue. The sharp tip may then degrade in the body or skin, opening up the inner microchannel of the microneedle 106 for exchange of fluids.

Base 102 may take various forms. In some embodiments, base 102 may take the form of i) a self-adhering patch *(e.g.,* e-skin or tattoo type device) with microneedles 106 on one side (i.e. the skin-contacting bottom side of the core actuation element in Figs. 1A and IB), as well as ii) other devices such as arm bands, cuffs, etc. In some embodiments, the same skin-contacting bottom side of base 102 that includes microneedles 106 may also include various biocompatible adhesives, although this is not required. While base 102 is depicted as including a reservoir 104, this is not meant to be limiting. As noted above, in some embodiments, a separate reservoir may be fluidly coupled with base 102, *e.g.,* via a conduit or port. In some such embodiments, base 102 may or may not include its own temporary reservoir.

In some embodiments, one or more microneedles 106 may be inserted or pierced into tissue 107 such that the tips 108 are positioned at respective sampling regions. In various embodiments, logic 90 may apply one or more types of energy (alone or in combination with other types of energy), such as heat, electricity, light, ultrasound, radio-frequency (RF) waves, etc. at one or more of the microneedles 106 to induce bioanalyte flow towards and/or through one or more microneedles. For example, one or more types of energy may be applied at or near the tips 108 of microneedles 106, *e.g.,* by individually-controllable needle-tip energy emitters 114 (only four of which are depicted in Figs. 1A and B but this is not meant to be limiting), to cause an increase in flow volume rate (*e.g.,* a local energy diffusion gradient) of blood and/or other fluids at each sampling region (i.e. tissue perfusion). This in turn may cause a corresponding increase in bioanalytes near the tips 108, thereby increasing capture of these bioanalytes.

In addition to increasing perfusion in tissue 107 proximate to microneedle tips 108 in order to enhance bioanalyte capture, various techniques are described herein for facilitating through-microneedle flow, *e.g.,* from microneedle tip 108, through an inner microchannel or lumen of microneedle 106, and into reservoir 104 (or other destinations). This may be accomplished using a variety of techniques, including heat gradients, iontophoresis, electrophoresis, dielectrophoresis, and so forth. For example, one or more individually-controllable needle-base energy emitters 112 (only four of which are depicted in Figs. 1A and B but this is not meant to be limiting) may be positioned at or near bases of microneedles 106 and may be operated, *e.g.,* by logic 90 via CAE 93, to apply one or more types of energy (*e.g.,* heat, electricity, light, ultrasound, RF waves, etc., alone or in combination) at microneedles 106 (*e.g.,* towards the bases of microneedles 106). In some embodiments, each needle-base energy emitter 112 may be wrapped entirely or at least partially around a respective base of a microneedle 106, although this is not required. In other embodiments, a needle-base energy emitter 112 may coat a portion of a base of a microneedle 106, or may otherwise be adjacent a base of the microneedle 106, *e.g.,* coupled to base 102.

In Fig. 1A, apparatus 100 is in the process of being affixed to tissue 107. In this example, microneedles 106 have pierced the skin surface 111 and epidermis 113, but have not yet pierced the epidermis-dermis junction (EDJ) 118 into the dermis 116. In Fig. 1B, apparatus 100 has been completely affixed to tissue 107. Tips 108 of microneedles 106 are now within closer proximity of vasculature 120 (*e.g.,* capillaries, arteries and/or veins) within dermis 116. Consequently, tips 108 of microneedles, as well as needle-tip energy emitters 114, are also in closer proximity to bioanalytes 122 of potential interest in vasculature 120.

In various embodiments, logic 90 may be adapted to (*e.g.,* automatically or in response to one or more commands received wirelessly from a remote computing device) operate at least a subset of the plurality of individually-controllable energy emitters (112 and 114) to apply energy at a first subset of the microneedles 106. In some embodiments, this applied energy may induce bioanalyte flow towards or through the first subset of the microneedles (i.e., the same microneedles at which the energy was applied). Additionally or alternatively, this applied energy may induce bioanalyte flow towards or through a second subset of the microneedles 106 that is different than the first subset.

For example, logic may operate first CAE 93₁ to cause needle-base energy emitters 112₁ and 112₂ to apply energy at respective bases of first microneedle 106₁ and second microneedle 106₂. Additionally or alternatively, logic may operate first CAE 93₁ to cause needle-tip energy emitters 114₁ and 114₂ to apply energy at respective tips 108 of first microneedle 106₁ and second microneedle 106₂. Depending on the type of energy applied, the manner of application (*e.g.,* applied at both the base and tip, applied only at the base or the tip, etc.), and the specific tissue structure (*e.g.* sweat gland, sebum gland, different skin layers) accessed by individual needle subsets, the applied energy may induce bioanalyte flow: (i) from tissue 107 towards tips 108 of microneedles 106₁ and 106₂; (ii) from tips 108 of microneedles 106₁ and 106₂ through microchannels of microneedles 106₁ and 106₂ into to reservoir 104; (iii) through microchannels of microneedles 106₁ and 106₂ from reservoir 104 towards tips 108 *(e.g.,* to clean the microchannels); (iv) away from tips 108 of microneedles 106₁ and 106₂ (*e.g.,* towards tips of other microneedles), and/or (v) enhance bioanalyte collection from various tissue structure sources through needle subsets. In some embodiments, some bioanalytes may be drawn towards/through microneedle 106 by a particular applied energy, while other bioanalytes or components may be driven away from microneedle 106 by the same applied energy.

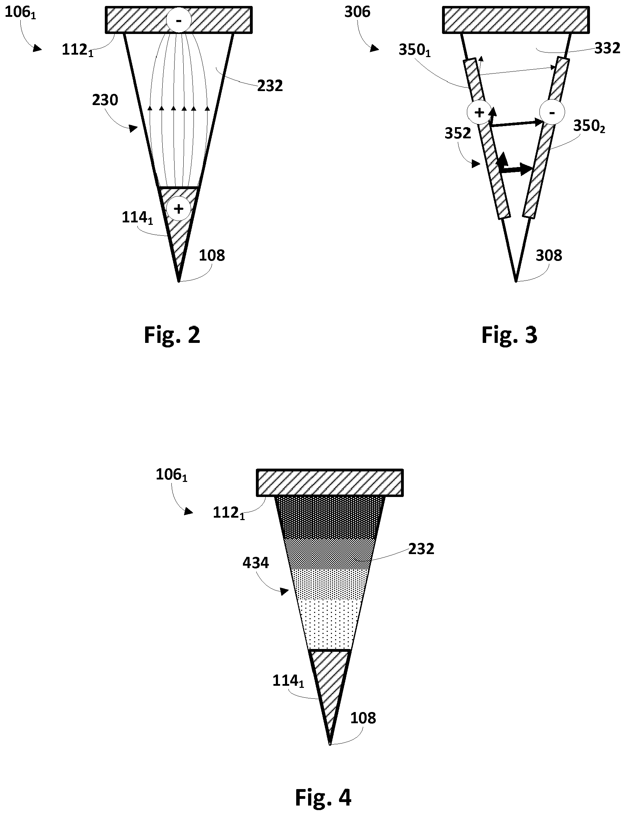

In some embodiments, the energy applied by energy emitters 112, 114 may take the form of electricity. For example, in some embodiments, a direct current (DC) electrical field may be generated to trigger electrophoresis to drive charged biomarker species such as metabolites (*e.g.,* Li, Na, K, Mg ions) at a higher rate in a direction of the electric field. In Fig. 2, for instance, first microneedle 106₁ is depicted with needle-base energy emitter 112₁ having a negative voltage(i.e. acting as a negative electrode) and needle-tip energy emitter 114₁ having a positive voltage(i.e. acting as a positive electrode). This polarity generates an electrical field 230 in a direction from tip 108 towards a base of microneedle 106₁, as indicated by the arrows. Positively-charged biomarkers (*e.g.,* ions) such as Li, Na, K, and/or Mg may be attracted to the negative electrode. Negatively charged biomarkers (*e.g.* Cl) may be driven to the positive electrode. In other cases, the needle-tip energy emitter 114₁ may have a negative voltage (i.e. acting as a negative electrode). Such a negative voltage would cause positively-charged biomarkers (*e.g.,* ions) such as Li, Na, K, and/or Mg to be attracted to the negative electrode at the tip from the surrounding tissue.

In some embodiments, electrodes that are directly in contact with an aqueous liquid *(e.g.,* in microchannel 232) may be kept at voltages below 2V. Greater voltages may trigger electrolysis and consequent gas formation. However, because microneedles 106 may be very small, it may still be possible to create a relatively strong electric field 230 even with such low voltages. In some embodiments, higher voltages may be achieved, *e.g.,* by screening the electrodes (112, 114) from fluid contained in microchannel 232. In various embodiments, electrical fields may be applied having strengths in the range of approximately 0.01 V/micron to approximately 10V/micron.

In other embodiments in which the applied energy takes the form of electricity, a DC voltage may be used to trigger iontophoresis. This in turn drives relatively small ions like H+ to cause an electrophoretic flow towards/ away from a microneedle 106 tip 108, and/or through the microneedle 106 in a desired direction. This electrophoretic flow may wash other uncharged bioanalytes along with the flow. An amount of captured bioanalytes may be controlled, for instance, by limiting current through the electrodes (i.e. 112 and 114).

In yet other embodiments in which the applied energy takes the form of electricity, an alternative current (AC) may be applied to create an AC electrical field. The AC electrical field may in turn drive movement of uncharged (but polarizable) bioanalytes, *e.g.,* using dielectrophoresis. Depending on a frequency of the AC electrical field, the polarizable bioanalytes may be moved in directions either toward or away from areas of high AC electrical field intensity. In some embodiments, frequencies may lie in the range of approximately 10Hz to approximately 1MHz. Microneedles 106 may be well-suited for this type of energy application.

Referring now to Fig. 3, two lateral energy emitters 350₁ and 350₂ (which may take the form of, and alternatively be referred to, as electrodes) may be positioned on opposite sides of a microneedle 306. By selectively operating energy emitters 350₁ and 350₂, it is possible to create a field gradient 352 indicated in Fig. 3. As the size (*e.g.,* the circumference and/or diameter) of the microneedle 306 decreases towards tip 308, the field intensity may increase (as indicated by arrow thickness in Fig. 3). Accordingly, an AC frequency may be selected to increase the speed of bioanalytes in either a direction from tip 308 to a base (for sampling) of microneedle 306, or from a base of microneedle 306 to tip 308 (for expelling fluid, e.g., to clean microchannel 332). In a variation of what is depicted in Fig. 3, in other embodiments, a needle-base energy emitter (e.g., 114) may take the form of a conventional voltage electrode. One or more concentric electrode rings (i.e. energy emitters) may be wrapped around the microneedle shaft to generate a gradient of AC electric field densities, e.g., within tissue generally, adjacent a microneedle tip, and/or within the microneedle itself. These AC electric field polarities or intensity gradients may accelerate sampling and movement of biomarkers in manners similar as described above related to electrophoresis and iontophoresis.

By using DC energy to selectively alternate one or more polarities between various electrodes (i.e. energy emitters) as described above, bioanalytes that are positively or negatively charged may be selectively analyzed on-the-fly. Additionally, bioanalytes may be driven by active forces, rather than solely by passive diffusion, providing a greater amount of control and selection. Furthermore, applying AC energy may facilitate active separation and/or up-concentration of various bioanalytes. For example, if a measurement of relatively small bioanalytes (e.g., electrolytes) is desired, bodily fluid sampled from tissue may be effectively "purified" of other components, e.g., by accelerating migration of the targeted analytes while other components are not accelerated or even deaccelerated. This is possible using AC energies because the direction of motion under dielectrophoresis is also a function of the size of the particle (e.g. the cell).

In other embodiments, the energy applied by energy emitters described herein *(e.g.,* 112, 114, 350) may take the form of heat. In various embodiments, the applied heat may induce one or more heat gradients towards/away from microneedle tips, and/or through microneedle microchannels towards a reservoir (for fluid/bioanalyte collection) or towards a microneedle tip (for cleaning).

Referring now to Fig. 4, which depicts a variation of the embodiment of Fig. 2, needle-tip energy emitter 114₁ and/or needle-base energy-emitter 112₁ may take the form of a heating element *(e.g.,* a thermo-element). In various embodiments, one or both of needle-base energy emitter 112₁ and needle-tip energy emitter 114₁ may be energized to provide heat (or cooled, as the case may be). When needle-tip energy emitter 114₁ is heated, the heat may stimulate bioanalyte diffusion in tissue near the tip of microneedle 106₁. In some embodiments, needle-base energy emitter 112₁ may be heated in conjunction with needle-tip energy emitter 114₁, *e.g.,* at a higher or lower temperature, to induce a heat gradient 434 through microneedle 106₁ and microchannel 232.

Suppose needle-tip energy emitter 114₁ is heated and needle-base energy emitter 112₁ is heated to a lower temperature (or not heated at all, or even cooled). Temperature gradient 434, which in this example may exhibit a decrease in temperature in a direction from tip 108 towards needle-base energy emitter 112₁, may drive heated tissue fluids (with relatively low densities and/or relatively high particle kinetic energies and diffusion speeds) through microneedle 106₁ in a direction from tip 108 towards needle-base energy emitter 112₁ (and hence, towards reservoir 104).

On the other hand, suppose a temperature at needle-tip energy emitter 114₁ is cooler than that at needle-base energy emitter 112₁ (*e.g.,* needle-base energy emitter 112₁ is heated to a higher temperature and needle-tip energy emitter 114₁ is not heated at all, or even cooled). Temperature gradient 434, which in this example may exhibit an increase in temperature in a direction from tip 108 towards needle-base energy emitter 112₁, may drive heated tissue fluids (with relatively low densities) to be drawn through microneedle 106₁ in a direction from needle-base energy emitter 112₁ (and upstream, from reservoir 104) towards tip 108. Such fluid flow towards the tip may be beneficial for cleaning the needle lumen.

While the heat-based examples above describe energy emitters 112 and 114 as being heating elements, this is not meant to be limiting. In some embodiments, other types of energy may be applied, *e.g.,* at needle-base energy emitter 112₁ and/or needle-tip energy emitter 114₁, to induce heat gradients towards/away from and/or through microneedles 106. For example, in some embodiments, needle-base energy emitters 112 and/or needle-tip energy emitters 114 may take the form of piezo-elements that may be used to generate (*e.g.,* high frequency, in some embodiments in a range of approximately 1 MHz to approximately 50 MHz) ultrasound, *e.g.,* using capacitive micromachined ultrasonic transducers (CMUT). For lower frequencies, more traditional ultrasound actuators may be employed. For example, it has been observed that application of ultrasound by a traditional ultrasound actuator having a 20 kHz frequency at 3W/cm² for two minutes increases skin temperature by several degrees. Such increase in skin temperature would suffice to facilitate diffusion of bioanalytes within tissue and/or induce heat gradients, both of which may lead to locally enhanced fluid flow (rate) through the tissue for enhanced bioanalyte sampling.

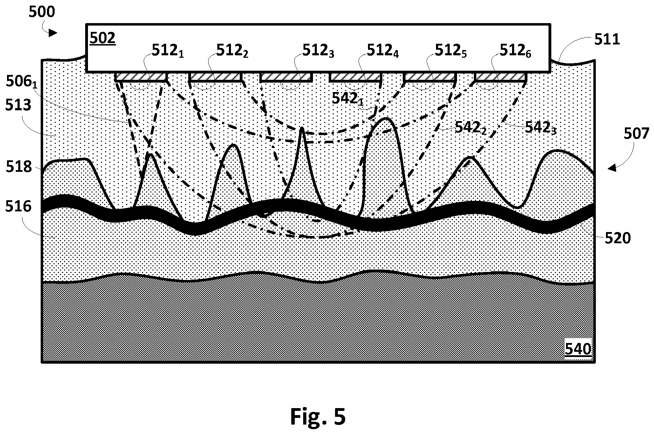

In other embodiments, RF energy may be applied to generate heat gradients and/or stimulate bioanalyte diffusion and enhanced flow rate in the tissue. Referring now to Fig. 5, an apparatus 500 configured with selected aspects of the present disclosure (many of which are not depicted in Fig. 5 for the sakes of brevity and clarity) is depicted affixed to tissue 507. Similar to Figs. 1A and 1B, tissue 507 is depicted in cross-section to illustrate various tissue layers, including epidermis 513, dermis 516, EDJ 518, and an additional layer, subcutaneous fat layer 540 (also referred to as the hypodermis). For the sake of clarity, only a single microneedle 506₁ is depicted in dashed lines. However, it should be understood that in various embodiments, more microneedles may be included with apparatus 500.

A plurality of RF-based energy-emitters 512₁₋₆ are also depicted, *e.g.,* adjacent to bases of microneedles (only first microneedle 506₁ indicated in Fig. 5). While six RF-based energy emitters 512₁₋₆ are depicted in Fig. 5, this is not meant to be limiting. More or less RF-based energy emitters 512 may be included. Additionally, while RF-based energy emitters 512 are depicted as being on or near bases of microneedles 506, this is not meant to be limiting. As is described elsewhere herein with regard to other embodiments, energy emitters may be coupled to microneedle bases, tips, along lengths of microneedles, or any combination thereof.

In this embodiment, energy emitters 512₁₋₆ may take the form of RF electrodes that may be operated to emit RF waves. In Fig. 5, the RF electrodes are depicted as having shapes similar to needle-base energy emitters 112. However, this is not required. In some embodiments, a given RF electrode may take the shape of a microneedle (*e.g.,* a hollow electrode with a sharp tip), in which case the RF electrode may have two functions: applying energy in the form of heat; and sample collection. In Fig. 5, third and fourth energy emitters 512₃ and 512₄ emit a first RF wave 542₁ (shown in dot-dot-dash lines). Second and fifth energy emitters 512₂ and 512₅ emit a second, wider RF wave 542₂ (shown in dot-dash-dash lines). First and sixth energy emitters 512₁ and 512₆ emit a widest third RF wave 542₃ (shown in dot-dash-dot lines).

The shape and depth of RF waves 542 emitted by energy emitters 512 (i.e. RF electrodes) may depend on the shape and/or size of the RF electrodes, as well as the applied voltage and frequency. It has been observed that RF energy may be used to increase skin temperature by several degrees using parameters such as the following: a frequency of 1 MHz; a voltage of 68 Vᵣₘₛ; an RF electrode diameter of 10 mm; an RF pulse repetition rate of 100 Hz; and an operating surface temperature of 36-52°C. However, these parameters are provided only as examples, and are not meant to be limiting.

In some embodiments, RF energy may be used increase tissue temperature in a manner that produces localized tissue lesions. Accordingly, RF energy can be applied and targeted to cause localized rupture of, for instance, vasculature 520, instead of using microneedles 506 to cause ablation. This may be achieved in some embodiments using relatively low voltages, such as approximately 60V or less, for relatively short pulse durations (*e.g.,* 200 ms). Non-limiting advantages of using RF energy to heat tissue, induce heat gradients, and/or perform ablation may include relatively low cost, the ability to selectively target specific sampling regions with RF energy, rupture of vasculature on demand, and so forth.

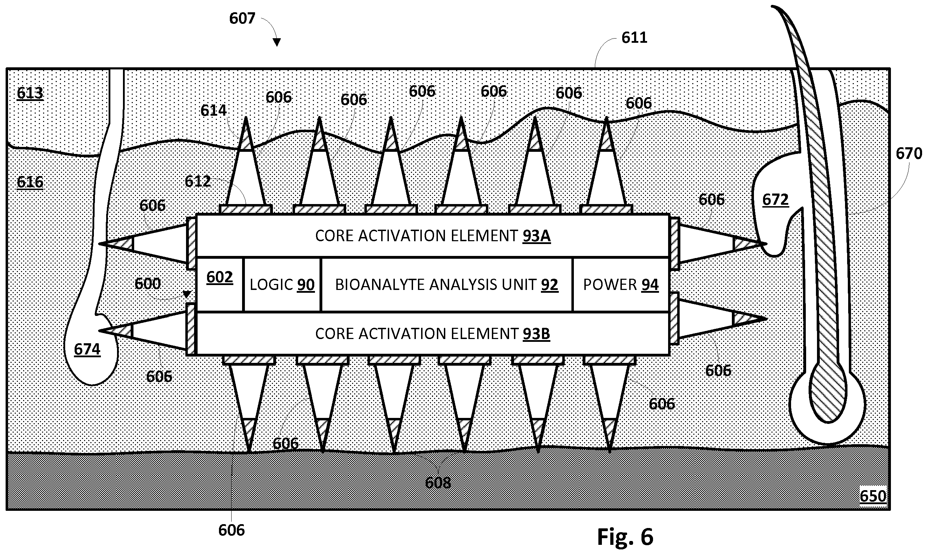

Fig. 6 depicts an alternative embodiment of an apparatus 600 that is similar to that depicted in Figs. 1A-B. However, in Fig. 6, rather than being a wearable device, apparatus 600 is an insertable device that has been inserted into tissue 607, *e.g.,* within dermis 616 (although it may be inserted at various other depths within tissue 607, such as epidermis 613). Additionally, unlike apparatus 100, apparatus 600 includes a plurality of microneedles 606 that extend from base 602 in multiple directions. In this example, microneedles 606 extend in opposite directions (both perpendicular from the skin surface) from two opposite sides of base 602.

Additionally, microneedles 606 may extend from base 602 in other directions, such as parallel to the skin surface 611. In Fig. 6, for example, laterally extending microneedles 606 penetrate, on the right, a sebum gland 672 associated with a hair follicle 670. On the left, laterally extending microneedles 606 penetrate a sweat gland 674. Accordingly, in addition to drawing fluids from/administering fluids to capillaries, as was described above, in embodiments such as that depicted in Fig. 6, fluids may be drawn from/injected into other anatomical components, such as sebum gland 672 and/or sweat gland 674. As described above, one or more types of energy *(e.g.,* heat, electricity, ultrasound, RF, light, etc.) may be applied (alone or in combination with other types of energy) to individual microneedles 606 and/or to subsets of microneedles 606. Applying energy to individual subsets of microneedles may allow for targeted sampling of different biological fluids (*e.g.* sweat, blood, lymph) which may need to be delivered /injected to different on-device analyzer modules available in the bioanalyte analysis unit (92).

In the embodiment of Fig. 6, each microneedle 606 includes a needle-base energy emitter 612 and a needle-tip energy emitter 614 (only one of which is labeled in Fig. 6 for the sake of clarity). However, this is not meant to be limiting. In various embodiments, some of the microneedles 606 may include both of these components, while other microneedles may include only a needle-base energy emitter 612 or a needle-tip energy emitter 614, but not both. In yet other embodiments, some microneedles 606 may include one or both of needle-base energy emitter 612 or needle-tip energy emitter 614, while other microneedles 606 may include neither. More generally, in any embodiment described herein, some or all microneedles may include one or more of a needle-base energy emitter, a needle-tip energy emitter, or other types of energy emitters described herein *(e.g.,* 350 in Fig. 3).

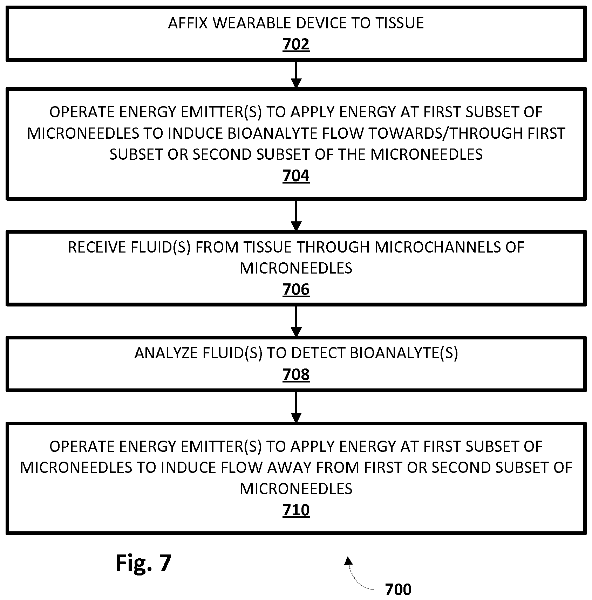

Fig. 7 depicts an example method 700 for improved sampling of various bioanalytes from bodily fluids using wearable and/or insertable devices. While operations of method 700 are depicted in a particular order, this is not meant to be limiting. In various embodiments, one or more operations may be added, omitted, and/or reordered.

At block 702, a wearable or insertable apparatus configured with selected aspects of the present disclosure may be affixed to *(e.g.,* inserted into) tissue of a patient, such as the patient's skin. In some embodiments, this affixing may include inserting a plurality of microneedles into the tissue. The apparatus may be adhered to the patient's tissue in various ways. In some embodiments, insertion of the microneedles into the tissue may itself anchor the apparatus to the patient's tissue. Additionally or alternatively, various biocompatible adhesives may be applied to an underside of the substrate to affix a wearable device to the patient's tissue. In some embodiments, an adhesive bandage or other suitable component (*e.g.,* cuff, band, etc.) may be used to affix the wearable device to the patient's tissue.

At block 704, one or more energy emitters (*e.g.,* 112, 114, 350, 612, 614) may be operated to apply various types of external energy in various manners to one or more subsets of the plurality of microneedles, including at individual microneedles. As described above, this applied energy may induce localized energy gradients and a bioanalyte flow through the tissue and/or towards microneedles and/or through microchannels of microneedles, *e.g.,* into a reservoir (for sampling). At block 706, as a result of the induced bioanalyte flow, one or more bodily fluids containing targeted bioanalytes may be received, *e.g.,* at the aforementioned conduit or port (which may lead to an integral reservoir, to a removable reservoir, or to a fluid line to a remote location) through microchannels of the microneedles.

At block 708, the one or more fluids may be analyzed, *e.g.,* by bioanalyte analysis unit 92, to detect one or more bioanalytes. In some embodiments, bioanalytes may be detected in terms of absolute quantities. In other embodiments, bioanalytes may be detected as being present in ratios, *e.g.,* compared to other bioanalytes and/or other substances. At optional block 710, one or more energy emitters may be operated to expel fluid through microchannels of one or more subsets of the plurality of microneedles, *e.g.,* by inducing fluid flow away from the one or more subsets of the microneedles, *e.g.,* to clean the microneedles.

In said the applied energy may comprise electricity that induces iontophoresis, electrophoresis, or dielectrophoresis through the first or second subset of the plurality of microneedles or towards tips of the first or second subset of the plurality of microneedles.

Further the method may comprise radio frequency energy or ultrasound energy that increases a temperature of tissue or creates a heat diffusion gradient that induces the bioanalyte flow.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements), etc.

As used herein in the specification and in the claims, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03. It should be understood that certain expressions and reference signs used in the claims pursuant to Rule 6.2(b) of the Patent Cooperation Treaty ("PCT") do not limit the scope.

## Claims

1. An apparatus (100, 500, 600) for sampling one or more bioanalytes in tissue (107, 507, 607), comprising:
a base (102, 502, 602) having one or more conduits adapted to receive fluid extracted from the tissue;
a plurality of microneedles (106, 306, 506, 606) fluidly coupled with the one or more conduits and adapted to be pierced into the tissue such that a tip (108, 308) of each microneedle is positioned in a respective sampling region of the tissue;
a plurality of individually-controllable energy emitters (112, 114, 350, 612, 614); and
logic (90) operably coupled with the plurality of individually-controllable energy-emitters, wherein the logic is adapted to operate a subset of the plurality of individually-controllable energy emitters to apply energy at a first subset of the plurality of microneedles to induce bioanalyte flow through tissue towards the first subset or a second subset of the plurality of microneedles, or through the first subset or a second subset of the plurality of microneedles.

2. The apparatus of claim 1, wherein the applied energy comprises electricity.

3. The apparatus of claim 2, wherein the electricity is direct current.

4. The apparatus of claim 2, wherein the electricity induces iontophoresis through the first or second subset of the plurality of microneedles.

5. The apparatus of claim 2, wherein the electricity induces iontophoresis in respective sampling regions towards the tips of the first or second subset of the plurality of microneedles.

6. The apparatus of claim 2, wherein the electricity induces electrophoresis or dielectrophoresis through the first or second subset of the plurality of microneedles.

7. The apparatus of claim 2, wherein the electricity induces electrophoresis or dielectrophoresis in respective sampling regions towards the tips of the first or second subset of the plurality of microneedles.

8. The apparatus of claim 2, wherein the electricity is alternating current.

9. The apparatus of claim 1, wherein at least one of the energy emitters is adjacent a proximal end of a microneedle of the plurality of microneedles.

10. The apparatus of claim 1, wherein at least one of the energy emitters is coupled to the tip of a microneedle of the plurality of microneedles.

11. The apparatus of claim 1, wherein the applied energy comprises heat that induces one or more heat gradients towards or through the first or second subset of the plurality of microneedles.

12. The apparatus of claim 1, wherein the applied energy comprises ultrasound.

13. The apparatus of claim 12, wherein the ultrasound induces one or more heat diffusion gradients towards or through the first or second subset of the plurality of microneedles.

14. The apparatus of claim 1, wherein the applied energy comprises radio-frequency energy.

15. A method for sampling bioanalytes in tissue, comprising:
removably affixing (702) a wearable or insertable device that includes a substrate and a plurality of microneedles (106, 306, 506, 606) to tissue (107, 507, 607) of a patient, wherein the applying includes inserting the plurality of microneedles into the tissue;
operating (704) a plurality of individually-controllable energy emitters (112, 114, 350, 612, 614) of the wearable or insertable device to apply energy at a first subset of the plurality of microneedles to induce bioanalyte flow towards or through the first subset or a second subset of the plurality of microneedles;
receiving (706) one or more fluids from the tissue through microchannels of the plurality of microneedles; and
analyzing (708) the one or more fluids to detect one or more bioanalytes.

## Patentansprüche

1. Vorrichtung (100, 500, 600) zur Probenentnahme eines oder mehrerer Bioanalyten in Gewebe (107, 507, 607), umfassend:
eine Basis (102, 502, 602) mit einer oder mehreren Leitungen, die angepasst sind, um aus dem Gewebe extrahierte Flüssigkeit aufzunehmen;
eine Vielzahl von Mikronadeln (106, 306, 506, 606), die flüssig mit der einen oder den mehreren Leitungen gekoppelt sind, und so angepasst sind, dass sie in das Gewebe durchgestochen werden, so dass eine Spitze (108, 308) jeder Mikronadel in einem jeweiligen Probenentnahmebereich des Gewebes positioniert ist;
mehrere individuell steuerbare Energieemitter (112, 114, 350, 612, 614); und
Logik (90), die funktionsfähig mit der Vielzahl von individuell steuerbaren Energieemittern gekoppelt ist, wobei die Logik angepasst ist, um eine Teilmenge der Vielzahl von individuell steuerbaren Energieemittern zu betreiben, um Energie an einer ersten Teilmenge der Vielzahl von Mikronadeln anzulegen, um einen Bioanalytenfluss durch Gewebe in Richtung der ersten Teilmenge oder einer zweiten Teilmenge der Vielzahl von Mikronadeln oder durch die erste Teilmenge oder eine zweite Teilmenge der Vielzahl von Mikronadeln zu induzieren.

2. Vorrichtung nach Anspruch 1, wobei die angelegte Energie Elektrizität umfasst.

3. Vorrichtung nach Anspruch 2, wobei die Elektrizität Gleichstrom ist.

4. Vorrichtung nach Anspruch 2, wobei die Elektrizität eine Iontophorese durch die erste oder zweite Teilmenge der Vielzahl von Mikronadeln induziert.

5. Vorrichtung nach Anspruch 2, wobei die Elektrizität eine Iontophorese in jeweiligen Probenentnahmebereichen in Richtung der Spitzen der ersten oder zweiten Teilmenge der Vielzahl von Mikronadeln induziert.

6. Vorrichtung nach Anspruch 2, wobei die Elektrizität Elektrophorese oder Dielektrophorese durch die erste oder zweite Teilmenge der Vielzahl von Mikronadeln induziert.

7. Vorrichtung nach Anspruch 2, wobei die Elektrizität Elektrophorese oder Dielektrophorese in den jeweiligen Probenentnahmebereichen in Richtung der Spitzen der ersten oder zweiten Teilmenge der Vielzahl von Mikronadeln induziert.

8. Vorrichtung nach Anspruch 2, wobei die Elektrizität Wechselstrom ist.

9. Vorrichtung nach Anspruch 1, wobei mindestens einer der Energieemitter zu einem proximalen Ende einer Mikronadel der Vielzahl von Mikronadeln benachbart ist.

10. Vorrichtung nach Anspruch 1, wobei mindestens einer der Energieemitter an die Spitze einer Mikronadel der Vielzahl von Mikronadeln gekoppelt ist.

11. Vorrichtung nach Anspruch 1, wobei die angelegte Energie Wärme umfasst, die einen oder mehrere Wärmegradienten in Richtung oder durch die erste oder zweite Teilmenge der Vielzahl von Mikronadeln induziert.

12. Vorrichtung nach Anspruch 1, wobei die angelegte Energie Ultraschall umfasst.

13. Vorrichtung nach Anspruch 12, wobei der Ultraschall eine oder mehrere Wärmediffusionsgradienten in Richtung oder durch die erste oder zweite Teilmenge der Vielzahl von Mikronadeln induziert.

14. Vorrichtung nach Anspruch 1, wobei die angelegte Energie Hochfrequenzenergie umfasst.

15. Verfahren zur Probenentnahme von Bioanalyten in Gewebe, umfassend:
entfernbares Befestigen (702) einer tragbaren oder einführbaren Vorrichtung, die ein Substrat und mehrere Mikronadeln (106, 306, 506, 606) an Gewebe (107, 507, 607) eines Patienten enthält, wobei das Anlegen das Einführen der mehreren Mikronadeln in das Gewebe umfasst;
Betreiben (704) mehrerer individuell steuerbarer Energieemitter (112, 114, 350, 612, 614) der tragbaren oder einführbaren Vorrichtung, um Energie an einer ersten Teilmenge der Vielzahl von Mikronadeln anzulegen, um den Bioanalytenfluss zu oder durch die erste Teilmenge oder eine zweite Teilmenge der Vielzahl von Mikronadeln zu induzieren;
Empfangen (706) eines oder mehrerer Flüssigkeiten aus dem Gewebe durch Mikrokanäle der Vielzahl von Mikronadeln; und
Analysieren (708) der einen oder mehreren Flüssigkeiten, um einen oder mehrere Bioanalyten nachzuweisen.

## Revendications

1. Appareil (100, 500, 600) pour le prélèvement d'échantillons d'un ou plusieurs bioanalytes dans un tissu (107, 507, 607), comprenant:
une base (102, 502, 602) ayant un ou plusieurs conduits adaptés pour recevoir du fluide extrait du tissu;
une pluralité de microaiguilles (106, 306, 506, 606) couplées de manière fluidique avec les un ou plusieurs conduits et adaptées pour être pénétrées dans le tissu de sorte qu'une pointe (108, 308) de chaque microaiguille est positionnée dans une région de prélèvement d'échantillons respective du tissu;
une pluralité d'émetteurs d'énergie commandables individuellement (112, 114, 350, 612, 614); et
une logique (90) couplée de manière opérationnelle à la pluralité d'émetteurs d'énergie commandables individuellement, où la logique est adaptée pour faire fonctionner un sous-ensemble de la pluralité d'émetteurs d'énergie commandables individuellement afin d'appliquer de l'énergie à un premier sous-ensemble de la pluralité de microaiguilles pour induire un flux de bioanalytes à travers le tissu vers le premier sous-ensemble ou un deuxième sous-ensemble de la pluralité de microaiguilles, ou à travers le premier sous-ensemble ou un deuxième sous-ensemble de la pluralité de microaiguilles.

2. Appareil selon la revendication 1, dans lequel l'énergie appliquée comprend de l'électricité.

3. Appareil selon la revendication 2, dans lequel l'électricité est un courant continu.

4. Appareil selon la revendication 2, dans lequel l'électricité induit une iontophorèse à travers le premier ou le deuxième sous-ensemble de la pluralité de microaiguilles.

5. Appareil selon la revendication 2, dans lequel l'électricité induit une iontophorèse dans des régions de prélèvement d'échantillons respectives vers les pointes du premier ou du deuxième sous-ensemble de la pluralité de microaiguilles.

6. Appareil selon la revendication 2, dans lequel l'électricité induit une électrophorèse ou une diélectrophorèse à travers le premier ou le deuxième sous-ensemble de la pluralité de microaiguilles.

7. Appareil selon la revendication 2, dans lequel l'électricité induit une électrophorèse ou diélectrophorèse dans les régions de prélèvement d'échantillons respectives vers les pointes du premier ou du deuxième sous-ensemble de la pluralité de microaiguilles.

8. Appareil selon la revendication 2, dans lequel l'électricité est un courant alternatif.

9. Appareil selon la revendication 1, dans lequel au moins l'un des émetteurs d'énergie est adjacent à une extrémité proximale d'une microaiguille de la pluralité de microaiguilles.

10. Appareil selon la revendication 1, dans lequel au moins l'un des émetteurs d'énergie est couplé à la pointe d'une microaiguille de la pluralité de microaiguilles.

11. Appareil selon la revendication 1, dans lequel l'énergie appliquée comprend de la chaleur qui induit un ou plusieurs gradients de chaleur vers ou à travers le premier ou le deuxième sous-ensemble de la pluralité de microaiguilles.

12. Appareil selon la revendication 1, dans lequel l'énergie appliquée comprend des ultrasons.

13. Appareil selon la revendication 12, dans lequel les ultrasons induisent une ou plusieurs gradients de de diffusion de chaleur vers ou à travers le premier ou le deuxième sous-ensemble de la pluralité de microaiguilles.

14. Appareil selon la revendication 1, dans lequel l'énergie appliquée comprend une énergie radiofréquence.

15. Procédé de prélèvement d'échantillons de bioanalytes dans un tissu, comprenant:
apposer de manière amovible (702) un dispositif portable ou insérable qui comprend un substrat et une pluralité de micro-aiguilles (106, 306, 506, 606) au tissu (107, 507, 607) d'un patient, où l'application comprend l'insertion de la pluralité de micro-aiguilles dans le tissu;
faire fonctionner (704) une pluralité d'émetteurs d'énergie commandables individuellement (112, 114, 350, 612, 614) du dispositif portable ou insérable pour appliquer de l'énergie à un premier sous-ensemble de la pluralité de microaiguilles pour induire un flux de bioanalytes vers ou à travers le premier sous-ensemble ou un deuxième sous-ensemble de la pluralité de microaiguilles;
recevoir (706) un ou plusieurs fluides du tissu à travers des microcanaux de la pluralité de microaiguilles; et
analyser (708) les un ou plusieurs fluides pour détecter un ou plusieurs bioanalytes.
